Europäisches Patentamt

European Patent Office

Office européen des brevets

(19)

(11) Publication number: **0 443 573 A2**

# (12) EUROPEAN PATENT APPLICATION

(21) Application number: **91102531.0**

(22) Date of filing: **21.02.91**

(51) Int. Cl.⁵: **C07K 5/02, A61K 37/02, C07K 1/00**

(30) Priority: **23.02.90 US 484135**

(43) Date of publication of application:
**28.08.91 Bulletin 91/35**

(84) Designated Contracting States:
**AT BE CH DE DK ES FR GB GR IT LI LU NL SE**

(71) Applicant: **BIO-MEGA INC.**
**2100 Rue Cunard**
**Laval Québec H7S 2G5(CA)**

(72) Inventor: **Anderson, Paul Cates**
**4130 Edward Higgins**
**Pierrefonds, Quebec, H8Y 3M8(CA)**
Inventor: **Moss, Neil**
**3 Place Bellerive, Apt. 2212**
**Laval, Quebec, H7V 1B2(CA)**
Inventor: **Poupart, Marc-André**
**101 Aimé-Séguin**
**Laval, Quebec, H7M 1B3(CA)**
Inventor: **Yoakim, Christiane**
**63 Gianchetti**
**Laval, Quebec, H7G 4T5(CA)**

(74) Representative: **Laudien, Dieter, Dr. et al**
**Boehringer Ingelheim GmbH, Abteilung**
**Patente**
**W-6507 Ingelheim am Rhein(DE)**

(54) HIV protease inhibitors containing derived amino acids.

(57) Disclosed herein are compounds of the general formula X-A-B-C'-Y wherein X is lower alkanoyl optionally substituted with one or two hydroxyls, A is an amino acid residue having a lower alkyl side chain, e.g. Ala or Val, B is a non-peptide linking unit, e.g. statyl or PheΨ[CH₂NH]Leu, C' is an amino acid residue of α-amino-β-cycloalkylpropionic acid, glutamic acid or α-aminoadipic acid, or a related derivative thereof, and Y is alkoxy, cycloalkoxy, a substituted alkoxy or a substituted amino group, e.g. cycloalkylalkoxy, arylalkoxy, alkylamino, (cycloalkyl)alkylamino or arylalkylamino. The compounds inhibit the activity of human immunodeficiency virus (HIV) protease and interfere with HIV induced cytopathogenic effects in human cells. These properties render the compounds useful for combating HIV infections.

EP 0 443 573 A2

Field of the Invention

This invention relates to compounds exhibiting activity against particular retroviruses, to processes for producing the compounds, to pharmaceutical preparations thereof, and to the use of the compounds for the manufacture of a medicament for combating infections caused by the retroviruses.

Background of the Invention

During the last ten years, increasing attention has been focused on retroviruses. These viruses cause a variety of diseases in vertebrates, the most insidious to humans being immunodeficiencies and cancers. In 1983, a retrovirus, known as human immunodeficiency virus type 1 (HIV-1), was established as a causative agent for acquired immune deficiency syndrome (AIDS). This virus has become a pestilence of alarming proportion. More recently, the closely related virus, human immunodeficiency virus type 2 (HIV-2) has been identified as a second causitive agent of AIDS. (Hereinafter, the term "HIV" is meant to include both HIV-1 and HIV-2 and any mutants thereof.)

Presently, several compounds are being evaluated in the clinic as possible therapeutic agents for AIDS. Another compound, 3-deoxythymidine (known also as zidovudine or AZT), has been shown in the clinic to decrease mortality and the frequency of opportunistic infections in AIDS patients. This latter compound is being used to manage certain patients with symptomatic HIV infections. However, in spite of some recent progress, the need for an effective therapy for AIDS still exists. For recent reviews, see R.A. Weiss in "Molecular Basis of Virus Disease", Symposium of the Society for General Microbiology, Vol. 40, Eds. W.C. Russel and J.W. Almond, University Press, Cambridge, UK, 1987, pp 167-192, and R.C. Gallo and L. Montagnier, Scientific American, 259, (4), 40 (1988).

One approach to finding agents having anti-HIV activity is to inhibit the action of HIV-encoded enzymes. This manner of inhibition interferes with the replication and propagation of the virus. Such an approach has been applied successfully in a search for inhibitors of the viral encoded enzyme, reverse transcriptase (RT). More explicitly, the previously noted zidovudine was found to inhibit RT which is required for viral replication. Subsequently, zidovudine was developed as an anti-HIV agent. Still more recently, this approach has been investigated using another HIV-encoded enzyme known as HIV protease as the target enzyme. In one instance, pepstatin A was found to inhibit part of the intracellular processing of the HIV gag proteins. See, S. Seelmeier et al., Proc. Natl. Acad. Sci. USA, 85, 6612 (1988). However, the development of pepstatin A as an anti-HIV agent seems improbable in view of its multiple activities. Subsequently, M.L. Moore et al., Biochem. Biophys. Res. Comm., 159, 420 (1989) and G.B. Dreyer et al., Proc. Natl. Acad. Sci. USA, 86, 9752 (1989) reported on investigations showing the inhibition of HIV protease by hexapeptide and heptapeptide analogs. A.D. Richards et al., FEBS Letters, 247, 113 (1989), also have reported that acetyl-pepstatin and an octapeptide analog inhibit HIV protease in vitro. Similarly, in vitro inhibition of HIV protease activity by modified peptide substrates, based on amino acid sequences of six to ten amino acids of the HIV polyprotein, has been reported by S. Billich et al., J. Biol. Chem., 263, 17905 (1988) and by M. Kotler et al., Proc. Natl. Acad. Sci. USA, 85, 4185 (1988). The recent publication of patent applications, typically B. Weidmann, UK patent application 2,203,740, published October 26, 1988, I.S. Sigal et al., European patent application 337,714, published October 18,1989, and D.J. Kempf et al., PCT patent application WO 89/10752, published November 16, 1989, disclosing peptide-like compounds with retroviral protease inhibition, is indicative of the increasing interest in this area.

The present application discloses a group of compounds which are potent inhibitors of HIV protease. Moreover, a capacity to inhibit HIV induced cytopathogenic effects in human cells has been demonstrated for many of the compounds. Such properties, together with the attributes of a relatively selective action and an apparent lack of toxicity, renders the compounds useful as agents for combating HIV infections.

The compounds of this invention can be considered to have an incidental relationship to peptide derivatives since they have amino acid residues and/or derived amino residues incorporated into their structure and as such have a partial structural resemblance to pepstatin A and the previously noted peptide analogs. The present compounds also possess a partial structural resemblance to peptide derivatives reported to be renin inhibitors; for instance, see D.F. Veber et al., European patent application 77,028, published April 20, 1983, R.E. Ten Brink, PCT patent application WO 87/02986, published May 21, 1987, P. Raddatz Australian patent application 73833/87, published December 17, 1987, J.P. Hudspeth et al., US patent 4,743,585, issued May 10, 1988, W.J. Greenlee et al., European patent application 273,696, published July 6, 1988, and A. Wagner et al., Australian patent application 76241/87, published February 4, 1988. The remaining structural features and differences in biological profiles distinguish the prior art compounds from the present peptide derivatives, notwithstanding the existence of broad generic disclosures, such as found in the previously noted patent applications of Ten Brink and Sigal et al., encompassing an almost infinite number of compounds.

## Summary of the Invention

The compounds of this invention are represented by formula 1

X-A-B-C'-Y    $\underline{1}$

wherein X is

(i) a lower alkanoyl,

(ii) a lower alkanoyl monosubstituted with a hydroxy, or

(iii) an alkanoyl, containing three to six carbon atoms, disubstituted with hydroxy, each hydroxy being on different carbon atoms thereof;

A is NHCH($R^1$)C(O) wherein $R^1$ is lower alkyl,

B is NHCH($R^2$)-Q wherein $R^2$ is

(i) lower alkyl,

(ii) lower cycloalkyl,

(iii) (lower cycloalkyl)methyl,

(iv) phenyl or phenyl monosubstituted with hydroxy, lower alkyl or lower alkoxy, or

(v) benzyl or benzyl monosubstituted on the aromatic portion thereof with hydroxy, halo, lower alkyl or lower alkoxy, and

Q is CH(OH)$CH_2$C(O) or $CH_2$NHCH($R^3$)C(O) wherein $R^3$ has the same meaning as defined hereinabove for $R^2$,

C' is NHCH($R^4$)C(W) wherein $R^4$ is (lower cycloalkyl)methyl, $CR^5R^6CR^7R^8$C(O)-T or $CR^5R^6CR^7R^8CR^9R^{10}$C(O)-T wherein $R^5$ to $R^{10}$, inclusive, are each independently a hydrogen or lower alkyl and T is hydroxy or lower alkoxy, and W is oxo or thioxo;

Y is OR$^{11}$ wherein $R^{11}$ is

(i) (1-10C)alkyl,

(ii) lower cycloalkyl,

(iii) (lower cycloalkyl)-(lower alkyl), or

(iv) phenyl(lower alkyl) or phenyl(lower alkyl) monosubstituted on the aromatic portion thereof with hydroxy, lower alkyl, lower alkoxy, halo, $SO_2,NH_2$, or S(O)$_n$R$^{12}$ wherein n is an integer from zero to two and $R^{12}$ is lower alkyl; or

Y is NR$^{13}$R$^{14}$ wherein $R^{13}$ is hydrogen or lower alkyl and $R^{14}$ has the same meaning as defined hereinabove for $R^{11}$; or a therapeutically acceptable salt thereof.

A preferred group of the compounds of this invention is represented by formula 1 wherein

X is as defined hereinabove;

A is Ala, Abu, Val, Nva, Leu Ile, Nle or Tbg;

B is NHCH($R^2$)-Q wherein $R^2$ is lower alkyl, (lower cycloalkyl)methyl, benzyl, (4-hydroxyphenyl)methyl or (4-methoxyphenyl)methyl and Q is CH(OH)$CH_2$C(O) or $CH_2$NHCH($R^3$)C(O) wherein $R^3$ is lower alkyl, (lower cycloalkyl)methyl or phenyl;

C' is NHCH($R^4$)C(W) wherein $R^4$ is cyclopropylmethyl, $CH_2CH_2$C(O)OH, $CH_2CH(CH_3)$C(O)OH, CH-($C_2H_5$)$CH_2$C(O)OH, $CH_2CH_2$C(O)OCH$_3$, $CH_2CH(CH_3)$C(O)OCH$_3$, CH($CH_3$)CH($CH_3$)C(O)OCH$_3$, $CH_2CH_2CH_2$C(O)OH or $CH_2CH_2CH_2$C(O)OCH$_3$ and W is oxo or thioxo; and

Y is OR$^{11}$ wherein $R^{11}$ is

(i) (1-10C)alkyl,

(ii) cyclopropyl, cyclopentyl or cyclohexyl,

(iii) (lower cycloalkyl)methyl, (lower cycloalkyl)ethyl or (lower cycloalkyl)propyl,

(iv) phenyl(lower alkyl) or phenyl(lower alkyl) substituted at position 4 of the phenyl with hydroxy, lower alkoxy, fluoro, chloro or lower alkylthio; or

Y is NR$^{13}$R$^{14}$ wherein $R^{13}$ is hydrogen or methyl and $R^{14}$ has the same meaning as defined in the last instance for $R^{11}$;

or a therapeutically acceptable salt thereof.

A more preferred group of the compounds is represented by formula 1 wherein X is acetyl, 1-oxopropyl, 1-oxobutyl, 3-methyl-1-oxobutyl, 3,3-dimethyl-1-oxobutyl, 2-ethyl-1-oxobutyl, 2-hydroxy-1-oxopropyl,3-hydroxy-1-oxopropyl or 2,3-dihydroxy-1-oxopropyl; A is Val, Nva, Leu, Ile, Nle or Tbg; B is NHCH($R^2$)CH-(OH)$CH_2$C(O) wherein $R^2$ is 1-methylpropyl, 2-methylpropyl, 1,1-dimethylethyl, cyclopropylmethyl, cyclohexylmethyl, benzyl, (4-hydroxyphenyl)methyl or (4-methoxyphenyl)methyl, or B is NHCH($R^2$)$CH_2$NHCH($R^3$)C-

EP 0 443 573 A2

(O) wherein $R^2$ is as defined in the last instance and $R^3$ is 1-methylethyl, 1-methylpropyl, 2-methylpropyl, 1,1-dimethylethyl, 2,2-dimethylpropyl, cyclopropylmethyl, cyclohexylmethyl or phenyl; C' is Cpa, Glu, Glu-(OMe), Adp or Adp(OMe); and Y is methoxy, ethoxy, hexyloxy, octyloxy, cyclopropyloxy, 3-cyclopropylpropoxy, 2-cyclohexylethoxy, 3-cyclohexylpropoxy, 2-phenylethoxy, 2-(4-fluorophenyl)ethoxy, 2-[4-(methylthio)phenyl]ethoxy, 3-phenylpropoxy, 3-(4-hydroxyphenyl)propoxy, 3(4-propoxyphenyl)propoxy, 3-(4-fluorophenyl)propoxy, 3-(4-chlorophenyl)propoxy, 3-[4-(methylthio)phenyl]propoxy, methylamino, 3-cyclopropylpropylamino, 2-cyclohexylethylamino, 3-cyclohexylpropylamino, 2-phenylethylamino, 2-(4-fluorophenyl)ethylamino, 2-[4-(methylthio)phenyl]ethylamino, 3-phenylpropylamino, 3-(4-hydroxyphenyl)-propylamino, 3-(4-methoxyphenyl)propylamino, 3-(4-propoxyphenyl)propylamino, 3-(4-fluorophenyl)-propylamino, 3-(4-chlorophenyl)propylamino or 3-[4-(methylthio)phenyl]propylamino; or a therapeutically acceptable salt thereof.

A most preferred group of the compounds is represented by formula 1 wherein X is acetyl, 1-oxopropyl, 3,3-dimethyl-1-oxobutyl, 2-ethyl-1-oxobutyl, 2(S)-hydroxy-1-oxopropyl or 2(S)-3-dihydroxy-1-oxopropyl; A is Val or Tbg, B either is NHCH($R^2$)CH(OH)CH$_2$C(O) wherein $R^2$ is 1-methylpropyl, 2-methylpropyl, butyl, cyclopropylmethyl or cyclohexylmethyl, or B is NHCH($R^2$)CH$_2$,NHCH($R^3$)C(O) wherein $R^2$ is benzyl and $R^3$ is 2-methylpropyl or cyclopropylmethyl, the two asymmetric centers of the last said radical having the (S) chiral configuration; C' is Cpa, Glu or Glu(OMe) and Y is 2-phenylethoxy, 3-phenyl-propoxy, 2-phenylethylamino, 2-(4-fluorophenyl)ethylamino, 2-[4-(methylthio)phenyl]ethylamino, 3-phenylpropylamino, 3-(4-hydroxyphenyl)propylamino, 3-(4-propoxyphenyl)propylamino or 3-[4-(methylthio)phenyl]propylamino; or a therapeutically acceptable salt thereof.

Included within the scope of this invention is a pharmaceutical composition for treating HIV infections in a human comprising a compound of formula 1, or a therapeutically acceptable salt thereof, and a pharmaceutically acceptable carrier.

The scope of the invention includes as well the use of said compounds for the manufacture of a medicament for treating HIV infections in a human, comprising an effective amount of the compound of formula 1, or a therapeutically acceptable salt thereof.

Also included within the scope is the use of said compounds for the manufacture of a medicament for protecting human cells against HIV pathogenesis, comprising an anti-HIV effective amount of a compound of formula 1, or a therapeutically acceptable salt thereof.

Processes for preparing the compounds of formula 1 are described hereinafter.


Details of the Invention


GENERAL

The term "residue" with reference to an amino acid means a radical derived from the corresponding $\alpha$-amino acid by eliminating the hydroxyl of the carboxy group and one hydrogen of the $\alpha$-amino group.

In general, the abbreviations used herein for designating the amino acids and the protective groups are based on recommendations of the IUPAC-IUB Commission of Biochemical Nomenclature, see European Journal of Biochemistry, 138, 9 (1984). For instance, Val, Glu, Tyr, Ala, Ile, Asp, Phe, Leu and Gly represent the residues of L-valine, L-glutamic acid, L-tyrosine, L-alanine, L-isoleucine, L-aspartic acid, L-phenylalanine, L-leucine and glycine, respectively. The symbol "Abu" represents the residue of 2(S)-aminobutyric acid. The symbol "Adp" represents the residue of 2(S)-aminoadipic acid. The symbols "Cpa" and "Cha" represent the residues of 2(S)-amino-3-cyclopropylpropionic acid (L-cyclopropylalanine) and 2(S)-amino-3-cyclohexylpropionic acid (L-cyclohexylalanine), respectively. The symbols "Nle", "Nva" and "Tbg" represent the residues of 2(S)-aminohexanoic acid (L-norleucine), 2(S)-aminopentanoic acid (L-norvaline) and 2-(S)-amino-3,3-dimethylbutyric acid, respectively. The symbol "Tyr(Me)" represents the residue of $O^4$-methyltyrosine. The symbol "Glu(OMe)" represents the residue of $O^5$-methyl hydrogen glutamate. The symbol "4F-Ph" represents the residue of 2(S)-amino-3-(4-fluorophenyl)propanoic acid.

The symbol "Ψ[CSNH]" used between the three letter representations of two amino acid residues means that the normal amide bond between those residues has been replaced with a thioamide bond.

The term "lower alkyl" as used herein, either alone or in combination with a radical, means straight chain alkyl radicals containing one to six carbon atoms and branched chain alkyl radicals containing three to four carbon atoms and includes methyl, ethyl, propyl, butyl, hexyl, 1-methylethyl, 1-methylpropyl, 2-methylpropyl and 1,1-dimethylethyl.

The term "(1-10C)alkyl" as used herein means a straight chain or branched chain alkyl radical containing one to ten carbon atoms and includes, for example, methyl, ethyl, hexyl, octyl, decyl, 1-methylpropyl and 4-methylpentyl.

4

The term "lower alkanoyl" as used herein means straight chain 1-oxoalkyl radicals containing from one to six carbon atoms and branched chain 1-oxoalkyl radicals containing three to six carbon atoms and includes acetyl, 1-oxopropyl, 3-methyl-1-oxobutyl, 3,3-dimethyl-1-oxobutyl and 2-ethyl-1-oxobutyl.

The term "lower cycloalkyl" as used herein, either alone or in combination with a radical, means saturated cyclic hydrocarbon radicals containing from three to six carbon atoms and includes cyclopropyl, cyclobutyl, cyclopentyl and cyclohexyl.

The term "lower alkoxy" as used herein means straight chain alkoxy radicals containing one to six carbon atoms and branched chain alkoxy radicals containing three to four carbon atoms and includes methoxy, ethoxy, propoxy, hexoxy, 1-methylethoxy, butoxy and 1,1-dimethylethoxy. The latter radical is known commonly as tertiary-butyloxy.

The term "halo" as used herein means a halo radical selected from bromo, chloro, fluoro or iodo.

The symbol "Boc" represents 1,1-dimethylethoxycarbonyl, known commonly as tertiary-butyloxycarbonyl. The symbol "Ph" represents a phenyl radical. Other symbols include Ac for acetyl, "4F-Ph" for 4-fluorophenyl, "4HO-Ph" for 4-hydroxyphenyl, "4MeS-Ph" for 4-(methylthio)phenyl, "4PrO-Ph" for 4-propoxyphenyl and "4NH$_2$SO$_2$-Ph" for 4-(aminosulfonyl)phenyl.

With reference to the symbol B of general formula 1, the radical "-NHCH(R$^2$)-Q-" wherein R$^2$ is as defined hereinabove and Q is CH(OH)CH$_2$C(O) represents the radical derived from the amino acid known as statine (i.e. 4(S)-amino-3(S)-hydroxy-6-methylheptanoic acid) and its close analogs. The radical is derived by eliminating the hydroxyl of the carboxy group and one hydrogen of the amino group of the corresponding γ-amino acid. Each such radical has two chiral centers and thus can exist in various optically active or optically inactive forms. All forms are included for the compounds of formula 1 and for the appropriate intermediates therefor, the 4(S)-amino-3(S)-hydroxy enantiomers being preferred. The requisite 4-amino-3-hydroxy pentanoic acids for preparing the synthon to incorporate the radical into the compound of formula 1 can be prepared by methods described by D.H. Rich and E.T.O. Sun, J. Med. Chem., 23, 27 (1980), and references therein.

The term "Sta" represents the radical -NHCH(2-methylpropyl)CH(OH)CH$_2$C(O)-, derived from statine. The term "ACPPA" represents the radical NHCH(cyclopropylmethyl)CH(OH)CH$_2$C(O)-, derived from 4-amino-5-cyclopropyl-3-hydroxypentanoic acid. The term "ACHPA" represents the radical -NHCH-(cyclohexylmethyl)CH(OH)CH$_2$C(O)-, derived from 4-amino-5-cyclohexyl-3-hydroxypentanoic acid, and the term "AHPPA" represents the radical -NHCH(benzyl)CH(OH)CH$_2$,C(O)-,derived from 4-amino-3-hydroxy-5-phenylpentanoic acid. The 4(S)-amino-3(S)-hydroxy enantiomers of these last four embodiments are preferred. Unless designated otherwise by an antecedent such as (3R, 4R), the terms Sta, ACPPA, ACHPA and AHPPA represent their respective 4(S)-amino-3(S)-hydroxy enantiomers.

Also note that when B is the radical "NHCH(R$^2$)-Q" wherein R$^2$ is as defined hereinabove and Q is CH$_2$NHCH(R$^3$)C(O) wherein R$^3$ is as defined hereinabove, the radical is equivalent to two adjoining, corresponding amino acid residues (or derived amino acids residues) wherein the amide bond joining the two residues is reduced. According to convention, the latter radical can be expressed symbolically as two amino acid residues (in the three letter system) with the symbol " Ψ[CH$_2$,NH]" inserted between the designation of the two adjoining amino acid residues. In this instance, the radical can be derived from α-amino acids having the (R)- or preferably the (S)-chiral configuration for the α-carbon atom. Accordingly, for example, the peptide of formula 1 wherein X is acetyl, A is Val, B is NHCH(benzyl)CH$_2$NHCH(2-methylpropyl)C(O) with two (S)-asymmetric centers, C' is Glu and Y is 3-phenylpropylamino can be designated as Ac-Val-PheΨ[CH$_2$NH]Leu-Glu-NHCH$_2$CH$_2$CH$_2$Ph.

With reference to the compounds of formula 1, note that the asymmetric α-carbon atoms of the amino acid residues or derived amino acid residues represented by the symbols A and C' have the S configuration, whereas asymmetric carbon atoms, residing in the side chain of these amino acid or derived amino acid residues also may have the R configuration.

The term "coupling agent" as used herein means an agent capable of effecting the dehydrative coupling of a free carboxy group of one reactant with a free amino group of another reactant to form an amide bond between the reactants. The agents promote or facilitate the dehydrative coupling by activating the carboxy group. Descriptions of such coupling agents and activating groups are included in general textbooks of peptide chemistry; for instance, E. Schroder and K.L. Lubke, "The Peptides", Vol. 1, Academic Press, New York, N.Y., 1965, pp 2-128, and K.D. Kopple, "Peptides and Amino acids", W.A. Benjamin, Inc., New York, N.Y., 1966, pp 33-51. Examples of coupling agents are thionyl chloride, diphenylphosphoryl azide, dicyclohexylcarbodiimide, N-hydroxysuccinimide, or 1-hydroxybenzotriazole in the presence of dicyclohexylcarbodiimide. A very practical and useful coupling agent is (benzotriazol-1-yloxy)tris-(dimethylamino)phosphonium hexafluorophosphate, described by B. Castro et al., Tetrahedron Letters, 1219 (1975), see also D. Hudson, J. Org. Chem., 53, 617 (1988), either by itself or in the presence of 1-hydroxybenzotriazole.

The term "pharmaceutically acceptable carrier" as used herein means a non-toxic, generally inert vehicle for the active ingredient, which does not adversely affect the ingredient.

The term "effective amount" as used herein means a predetermined amount of the compound of this invention sufficient to be effective against HIV in vivo.

## PROCESS

The compounds of formula 1 can be prepared by processes which incorporate therein methods commonly used in peptide synthesis such as the classical solution coupling of amino acid residues and/or peptide fragments. Such methods are described, for example, by E. Schroder and K. Lubke, cited above, in the textbook series, "The Peptides: Analysis, Synthesis, Biology", E. Gross et al., Eds., Academic Press, New York, N.Y., 1979-1987, Volumes 1 to 8, and by J.M. Stewart and J.D. Young in "Solid Phase Peptide Synthesis", 2nd ed., Pierce Chem. Co., Rockford, IL, USA, 1984.

A common feature of the aforementioned processes for preparing the present compounds is the protection of any labile side chain groups of the reactants with suitable protective groups which will prevent a chemical reaction from occurring at that site until the protective group is ultimately removed. Usually also common is the protection of an $\alpha$-amino group on an amino acid or a fragment while that entity reacts at the carboxy group, followed by the selective removal of the $\alpha$-amino protective group to allow subsequent reaction to take place at that location.

Generally speaking, the preparation of the compound of formula 1 can be achieved by classical solution coupling techniques of the units A, B, C" and Y, or fragments thereof, C" having the same meaning as defined hereinabove for C' except that a side chain carboxy or ester (alkoxycarbonyl) group, if present, is replaced by a protected carboxy. In this manner, one obtains the key intermediate of formula H-A-B-C"-Y wherein A, B, C" and Y are as defined herein. Subsequent coupling with a coupling agent of the acid of formula X-OH wherein X is as defined herein with the preceeding key intermediate gives directly the corresponding compound of formula 1 when C" of the key intermediate does not bear a protected carboxy. In the instance where C" of the key intermediate does bear a protected carboxy, the coupling gives the corresponding intermediate of formula X-A-B-C"-Y which is then subjected to carboxy deprotection, or to carboxy deprotection and esterification, to give the corresponding compound of formula 1.

A practical preparation of the compounds of formula 1 involves the coupling by classical solution techniques of the units B and A, either stepwise or as an A-B-fragment, e.g. apg-A-B-OH wherein apg represents an $\alpha$-amino protective group, to the fragment H-C"-Y to obtain the key intermediate.

More explicitly with regard to the characterization of the unit C" and the aforementioned fragment H-C"-Y, C" represents the radical $NHCH(R^{4A})C(W)$ wherein $R^{4A}$ is (lower cycloalkyl)methyl, $CR^5R^6CR^7R^8C(O)-T^1$ or $CR^5R^6CR^7R^8CR^9R^{10}C(O)-T^1$ wherein $R^5$ to $R^{10}$, inclusive, are as defined hereinabove and $T^1$ is a carboxy protective group, for example, benzyloxy, cyclohexyloxy or 2,6-dichlorobenzyloxy, and W is oxo or thioxo. Accordingly, the fragment H-C"-Y can, depending on whether W is oxo or thio, contain an ester bond, a thioester bond, amide bond or a thioamide bond, respectively.

The fragment H-C"-Y is prepared by conventional processes. For instance, a convenient process for preparing the fragment when Y is a substituted amine, i.e. Y is $NR^{13}R^{14}$, wherein $R^{13}$ and $R^{14}$ are as defined herein, involves the formation of an amide bond between two reactants representing precursor portions for the amino acid unit C' and the amine unit Y, followed if required by the conversion of the amide bond to a thioamide bond. More specifically, an amino acid derivative of the formula apg-NH-CH($R^{4A}$)C(O)OH in which apg is an $\alpha$-amino protective group (e.g. Boc) and $R^{4A}$ is as defined herein is coupled, by means of a coupling agent, with the appropriate amine of formula $HNR^{13}R^{14}$ wherein $R^{13}$ and $R^{14}$ are as defined herein to give the corresponding protected amide of formula apg-NHCH($R^{4A}$)C(O)NR$^{13}$R$^{14}$, which on amino deprotection yields the corresponding second fragment containing the amide bond. When the corresponding second fragment containing a thioamide bond is desired, the last-named protected amide is reacted with an agent capable of converting an amide group to a thioamide group, e.g. phosphorus pentasulfide or preferably Lawesson's reagent [see U. Pederson et al., Tetrahedron, 38, 3267 (1982)], to obtain the corresponding protected thioamide of formula apg-NHCH($R^{4A}$)C(S)NR$^{13}$R$^{\overline{14}}$ which on amino deprotection gives the corresponding second fragment containing a thioamide bond.

The fragment of formula H-C"-Y wherein Y is alkoxy, cycloalkoxy, arylalkoxy, etc., i.e. fragments containing an ester bond, can be prepared by the same processes noted above for the corresponding fragment wherein Y is a substituted amine. However, the appropriate alcohol of formula $R^{11}OH$ is substituted for the amine of formula $HNR^{13}R^{14}$.

Turning to the unit B, as noted previously this unit can be either of two subunits, i.e. $NHCH(R^2)CH(OH)$-

$CH_2C(O)$ wherein $R^2$ is as defined herein or $NHCH(R^2)CH_2NHCH(R^3)C(O)$ wherein $R^2$ and $R^3$ are as defined herein. The first-mentioned subunit can be incorporated the the compound of formula 1 by coupling the N-protected derivative of the corresponding 4-amino-3-hydroxypentanoic acid [e.g. $Boc\text{-}NHCH(R_2)CH(OH)\text{-}CH_2C(O)OH$] with the appropriate fragment of formula $H\text{-}C''\text{-}Y$ in which $C''$ and $Y$ are as defined herein above, and thereafter coupling the resulting intermediate (e.g. $Boc\text{-}NHCH(R^2)CH(OH)CH_2C(O)\text{-}C''\text{-}Y$) with the remaining units A and X to obtain the corresponding compound of formula 1, after deprotection and esterification when required. The second-mentioned subunit, $NHCH(R^2)CH_2NHCH(R^3)C(O)$, can be incorporated by forming the linear framework of the compound of formula 1, or a fragment thereof, by a reductive alkylation between two sub-fragments, each sub-fragment containing a precursor portion of the B unit and at least one of the sub-fragments containing one or more of the remaining units of the desired final product, whereby the $CH_2,NH$ bond of the B unit is formed; for example, the reductive N-alkylation of the sub-fragment of formula $NH_2CH(R^3)C(O)\text{-}C''\text{-}Y$ with the sub-fragment $Boc\text{-}NHCH(R^2)CHO$ in the presence of sodium cyanoborohydride to give the corresponding $Boc\text{-}NHCH(R^2)CH_2NHCH(R^3)C(O)\text{-}C''\text{-}Y$. Subsequent coupling of the remaining units to the resulting fragment affords the corresponding compound of formula 1, after deprotection and esterification when required.

The compound of formula 1 of this invention can be obtained in the form of a therapeutically acceptable salt. In the instance where a particular peptide has a residue which functions as a base, examples of such salts are those with organic acids, e.g. acetic, lactic, succinic, benzoic, salicylic, methanesulfonic or p-toluenesulfonic acid, as well as polymeric acids such as tannic acid or carboxymethyl cellulose, and also salts with inorganic acids such as hydrohalic acids, e.g. hydrochloric acid, or sulfuric acid, or phosphoric acid. If desired, a particular acid addition salt is converted into another acid addition salt, such as a non-toxic, pharmaceutically acceptable salt, by treatment with the appropriate ion exchange resin in the manner described by R.A. Boissonnas et al., Helv. Chim. Acta, 43, 1849 (1960). In the instance where a particular peptide has one or more free carboxy groups, examples of such salts are those with the sodium, potassium or calcium cations, or with organic bases, for example, triethylamine or N-methylmorpholine.

In general, the therapeutically acceptable salts of the peptides of formula 1 are biologically fully equivalent to the peptides themselves.

## BIOLOGICAL ASPECTS

The HIV protease inhibiting properties and the cell protective effect against HIV pathogenesis of the compounds of formula 1, or a therapeutically acceptable salt thereof, can be demonstrated by biochemical, microbiological and biological procedures.

A particular useful procedure for demonstrating the HIV protease inhibiting properties of the compounds of formula 1 or their therapeutically acceptable salts is the "Recombinant HIV Protease HLPC Assay". The procedure is based on the capacity of the test compound to inhibit enzymatic cleavage by HIV protease of a decapeptide (the substrate) having an amino acid sequence which includes a known HIV protease cleavage site of a HIV polyprotein; see H.G. Krausslich et al., Proc. Natl. Acad. Sci. USA, 86, 807 (1989). Details of this assay together with the results obtained for exemplified compounds of formula 1 are described in the examples hereinafter.

The capacity of the compounds of formula 1 or their therapeutically acceptable salts to protect cells against HIV infection can be demonstrated by microbiological procedures for evaluating the inhibitory effect of a test compound on the cytopathogenicity of HIV in human T4 cell lines. Typical of such procedures are those described by S. Harada and N. Yamamoto, Jpn. J. Cancer Res. (Gann), 76, 432 (1985), and S. Harada et al., Science 229, 563 (1985). An assay based on the latter procedures is described in the examples hereinafter.

When a compound of this invention, or a therapeutically acceptable salt thereof, is used to combat HIV infections in a human, the peptide can be administered orally, topically or parenterally, in a vehicle comprising one or more pharmaceutically acceptable carriers, the proportion of which is determined by the solubility and chemical nature of the compound, the chosen route of administration and standard biological practice. For oral administration, the compound or a therapeutically acceptable salt thereof can be formulated in unit dosage forms such as capsules or tablets each containing a predetermined amount of the active ingredient, ranging from about 25 to 500 mg, in a pharmaceutically acceptable carrier. For topical administration, the compound can be formulated in a pharmaceutically acceptable vehicle containing 0.1 to 10 percent, preferably 0.5 to 5 percent, of the active agent. Such formulations can be in the form of a cream, lotion, sublingual tablet, or preferably a transdermal patch or buccal patch. For parenteral administration, the compound of formula 1 is administered by either intravenous, subcutaneous or intramuscular injection, in compositions with pharmaceutically acceptable vehicles or carriers. For administration by injection, it is preferred to use the compound in solution in a sterile aqueous vehicle which may also contain other solutes such as buffers or preservatives as well as sufficient quantities of pharmaceutically acceptable salts or of glucose to make the solution isotonic.

Suitable vehicles or carriers for the above noted formulations can be found in standard pharmaceutical texts, e.g. in "Remington's Pharmaceutical Sciences", 16th ed., Mack Publishing Company, Easton, Penn., 1980.

The dosage of the compound will vary with the form of administration and the particular active agent chosen. Furthermore, it will vary with the particular host under treatment. Generally, treatment is initiated with small dosages substantially less than the optimum dose of the peptide. Thereafter, the dosage is increased by small increments until the optimum effect under the circumstances is reached. In general, the compound is most desirably administered at a concentration level that will generally afford antivirally effective results without causing any harmful or deleterious side effects.

For oral administration, the compound or a therapeutically acceptable salt is administered in the range of 1.0 to 75 mg per kilogram of body weight per day, with a preferred range of 2.5 to 20 mg per kilogram. With reference to systemic administration, the compound of formula 1 is administered at a dosage of 10 mcg to 1000 mcg per kilogram of body weight per day, although the aforementioned variations will occur. However, a dosage level that is in the range of from about 50 mcg to 500 mcg per kilogram of body weight per day is most desirably employed in order to achieve effective results.

Although the formulations disclosed hereinabove are effective and relatively safe medications for treating HIV infections, the possible concurrent administration of these formulations with other antiviral medications or agents to obtain beneficial results is not excluded. Such other antiviral medications or agents include soluble CD4, zidovudine, dideoxycytidine, phosphonoformate, ribavarin or antiviral interferons (e.g. α-interferon or interleukin-2).

The following examples illustrate further this invention. Solution percentages or ratios express volume to volume relationship, unless stated otherwise. Temperatures are given in degrees Celsius. Proton nuclear magnetic resonance (NMR) spectra were obtained at 200 MHz. Abbreviations used in the examples include Boc: t-butyloxycarbonyl; BOP: (benzotriazol-1-yloxy)tris(dimethylamino)phosphonium hexafluorophosphate; Bzl: benzyl; DMF: dimethyl formamide; Et₂O: diethyl ether; EtOAc: ethyl acetate; EtOH: ethanol; HPLC: high performance liquid chromatography: MeOH: methanol; TFA: trifluoroacetic acid; THF: tetrahydrofuran; TLC: thin layer chromatography.

Example 1

Boc-Glu(OBzl)-NHCH$_2$CH$_2$CH$_2$Ph

(N$^\alpha$-Boc-protected fragment H-C''-Y wherein C'' is NHCH(R$^4$)C(W) wherein R$^4$ is CH$_2$CH$_2$C(O)-OBzl, W is oxo and Y is NHCH$_2$CH$_2$CH$_2$Ph)

N-methylmorpholine (3.3 mL, 5.5 mmol), the substituted amine 3-phenylpropylamine (0.78 mL, 5.5 mmol) and BOP (2.43 g, 5.5 mmol) were added to a solution of Boc-Glu(OBzl)OH (1.69 g, 5.0 mmol) in acetonitrile (50 mL). The mixture was stirred at room temperature (20-22°) for 1 h, concentrated under reduced pressure to one half of its initial volume, and then diluted with EtOAc (50 mL). The resulting mixture was washed successively with cold 1 M aqueous HCl (3 x 15 mL), saturated aqueous NaHCO₃ (2 x 15 mL) and a saturated aqueous solution of NaCl (20 mL). The organic layer was dried (Na₂SO₄) and concentrated under reduced pressure. The residue was dissolved in warm EtOAc. The solution was diluted with 150 mL of Et₂O. The latter solution was washed several times with H₂O and then with saturated aqueous solution of NaCl. The organic layer was dried (Na₂SO₄) and then concentrated under reduced pressure. The residue was crystallized from EtOAc-hexane to give the desired product as a white solid (1.43 g, 63%), suitable for use for the preparation of the compound of formula 1 in which Y is NHCH₂CH₂CH₂Ph. ¹H NMR (CDCl₃) δ 1.42 (9H, s), 1.81 (3H, complex m), 2.10 (1H, complex m), 2.42-2.55 (2H, complex m), 2.64 (2H, t, J = 7.1 Hz), 3.27 (2H, q, J = 6.8 Hz), 4.08 (1H, q, J = 5.6 Hz), 5.13 (2N, s), 5.17 (1H, broad m), 6.15 (1H, broad m), 7.15-7.35, (10H, complex m). ¹H NMR also indicated that the product contained 5 mole percent of hexamethylphosphoric triamide.

Other N$^\alpha$-Boc-protected fragments of formula H-C$^1$-Y in which C$^1$ is NHCH(R$^4$)C(W) wherein R$^4$ is as defined herein and W is oxo and Y is NR$^{13}$R$^{14}$ wherein R$^{13}$ and R$^{14}$ are as defined herein can be prepared by the procedure of this example by replacing 3-phenylpropylamine with the appropriate amine. For example, replacement with 2-phenylethylamine gives Boc-Glu(OBzl)-NHCH₂CH₂Ph.

Example 2

Boc-Glu-(OBzl)Ψ[CSNH]-NHCH$_2$CH$_2$CH$_2$Ph

(N$^\alpha$-Boc-protected fragment H-C''-Y wherein C'' is NHCH(R$^4$)C(W) wherein R$^4$ is CH$_2$CH$_2$C(O)-OBzl, W is thioxo and Y is NHCH$_2$CH$_2$CH$_2$Ph)

A stirred mixture of Boc-Glu(OBzl)-NHCH$_2$CH$_2$CH$_2$Ph(227 mg, 0.5 mmol), described in Example 1, and Lawesson's reagent, [2,4-bis(4-methoxyphenyl)-1,3,2,4-dithiaphosphetane 2,4-disulfide, 120 mg, 0.3 mmol], see K. Clausen et al., Chemica Scripta, 20, 14 (1982), in dry THF (5 ml) was heated at 60° for 15 min. The reaction solvent was evaporated under reduced pressure. The resulting yellow oil was subjected to chromatography on TLC grade silica gel (30 g), using EtOAc-hexane (3:7) as the eluent. Boc-Glu(OBzl)Ψ-[CSNH]-NHCH$_2$CH$_2$CH$_2$Ph was thus obtained as a colourless oil (202 mg, 86%). $^1$N NMR (CDCl$_3$) δ 1.42 (9H, s), 1.88-2.16 (4H, complex m), 2.31-2.59 (2H, m), 2.67 (2H, t, J = 7.3 Hz), 3.65 (2H, q, J = 6.7 Hz), 4.38 (1H, q, J = 6.1 Hz), 5.12 (2H, s), 5.53 (1H, d, J = 8.3 Hz), 7.15-7.34 (10H, m), 8.07 (1H, broad, m).

Other N$^\alpha$-Boc-protected fragments of formula H-C''-Y wherein C'' is NHCH(R$^4$)C(W) wherein R$^4$ is as defined herein and W is thioxo, and Y is NR$^{13}$R$^{14}$ wherein R$^{13}$ and R$^{14}$ are as defined herein can be prepared by selecting the appropriate reactants and following the procedure of this example.

Example 3

Ac-Val-ACHPA-Glu-NHCH$_2$CH$_2$Ph and Ac-Val-ACHPA-Glu-(OMe)-NHCH$_2$CH$_2$Ph

A) Boc-ACHPA-Glu(OBzl)-NHCH$_2$CH$_2$Ph

A solution of the protected fragment of formula H-C''-Y, Boc-Glu(OBzl)-NHCH$_2$CH$_2$Ph (1.32 g, 3 mmol) in 6N HCl/dioxane (20 mL) was stirred at room temperature (20-22°) under a nitrogen atmosphere for 20 min. The solvent was evaporated and the residue was dried in high vacuum. The solid was suspended in dry acetonitrile (30 mL). Under a nitrogen atmosphere, the stirred suspension was adjusted to pH 8 (wet pH paper) by the addition of N-methylmorpholine, then Boc-ACHPA-OH (946 mg, 3 mmol) and BOP (1.46 g, 3.3 mmol) were added to the mixture. The resulting solution was stirred at room temperature for 2 h. The mixture was poured into a saturated aqueous solution of NaCl. The aqueous solution was extracted twice with EtOAc. The combined organic extracts were washed successively with 5% aqueous HCl (two times), saturated aqueous NaHCO$_3$ (two times) and saturated aqueous NaCl. The extract was dried (Na$_2$SO$_4$) and concentrated to dryness. The residue was purified by chromatography on silica gel (20 g), using a gradient of 1 to 5% MeOH in chloroform as the eluent, to give Boc-ACHPA-Glu(OBzl)NHCH$_2$CH$_2$Ph as an oil (1.1 g, 57%).

B) Boc-Val-ACHPA-Glu(OBzl)-NHCH$_2$CH$_2$Ph

By following the procedure of section A) of this example but replacing Boc-Glu(OBzl)-NHCH$_2$CH$_2$Ph with the product of that section and replacing Boc-ACHPA-OH with Boc-Val-OH, then Boc-Val-ACHPA-Glu-(OBzl)-NHCH$_2$CH$_2$Ph was obtained in a quantitative yield.

C) Ac-Val-ACHPA-Glu(OBzl)-NHCH$_2$CH$_2$Ph

By following the procedure of section A) of this example but replacing Boc-Glu(OBzl)-NHCH$_2$CH$_2$Ph with the product of section B of this example and replacing Boc-ACHPA-OH with the acid of formula X-OH, acetic acid, then Ac-Val-ACHPA-Glu(OBzl)NHCH$_2$CH$_2$Ph was obtained.

D) Title compounds of this example

The product of section C) of this example was subject to hydrogenolysis as follows: The product (147 mg) was dissolved in MeOH (5 ml). Oxygen was removed from the solution with a stream of dry nitrogen. Under an atmosphere of nitrogen, a catalytic amount of 20% palladium hydroxide on charcoal (25 mg) was added to the solution. The mixture was shaken on a Parr hydrogenator under an atmosphere of hydrogen (50 psi) for 90 min. The mixture was filtered through a 45μm membrane and the filtrate was concentrated to dryness to afford Ac-Val-ACHPA-Glu-NHCH$_2$CH$_2$Ph as an oil (26.2 mg, 26%). $^1$H NMR (CDCl$_3$)) δ 1.0 (6H, 2d), 0.8-1.9 (21H, m), 1.5-1.7 (6H, m), 2.1 (3H, s), 2.3 (2H, m), 2.8 (2H, t), 3.5 (3H, m), 3.8 (2H, m), 4.0 (2H, m), 4.3 (1H, m), 7.7-7.8 (1H, m), 7.1-7.3 (5H, m). Mass spectrum: 589 (M+H)⁺. Amino acid analysis: Glu, 1.04; Val, 0.97; ACHPA, 0.82.

The latter product (13.1 mg) was dissolved in EtOAc (10 mL). A slight excess of diazomethane in dry Et₂O was added to the solution (as indicated by the mixture maintaining a yellow color). The mixture was evaporated to dryness. Trituration of the residual oil with Et₂O gave Ac-Val-ACHPA-Glu(OMe)NHCH₂CH₂Ph as a white solid (9.7 mg). Mass spectrum: 603 (M+H)⁺. Amino acid analysis: Glu, 1.04; Val, 0.96, ACHPA, 0.93.

Example 4

Ac-Tbg-ACHPA-Glu-NHCH₂CH₂Ph and Ac-Tbg-ACHPA-Glu(OMe)-NHCH₂CH₂Ph

A) Boc-Tbg-ACHPA-OH

A solution of Boc-ACHPA-OEt (1.09 g, 3.17 mmol) in 6N HCl/dioxane (30 mL) was stirred at room temperature under a nitrogen atmosphere for 15 min. The solvent was evaporated and the residue dried in high vacuum. The residue was suspended in dry acetonitrile (90 mL). Under a nitrogen atmosphere, the stirred suspension was adjusted to pH 8 (wet pH paper) by the addition of N-methylmorpholine, then Boc-NHCH[C(CH₃)₃]C(O)-OH (Boc-t-butylglycine, 732 mg, 3.17 mmol) and BOP (1.54 g, 3.48 mmol) were added to the mixture. The mixture was stirred at room temperature for 2 h. The mixture was poured into a saturated aqueous solution of NaCl. The aqueous solution was extracted twice with EtOAc. The combined organic extracts were washed successively with 5% aqueous HCl (two times), saturated aqueous NaHCO₃ - (two times) and saturated aqueous NaCl (two times). The extract was dried (Na₂SO₄) and evaporated to dryness. The residue was purified by chromatography on silica gel (30 g), using a gradient of 1 to 5% CH₃OH in chloroform as the eluent, to afford Boc-Tbg-ACHPA-OEt (1.0 g, 74%). The latter compound (1.0 g, 2.19 mmol) was dissolved in MeOH-H₂O (5:1, 30 mL). After the addition thereto of 1N aqueous NaOH (2.19 mL, 2.19 mmol), the solution was concentrated to remove the methanol, rendered acidic by the addition of 10% aqueous citric acid and extracted (three times) with EtOAc. The extract was washed with saturated aqueous NaCl (two times), dried (Na₂SO₄) and evaporated to dryness to yield Boc-Tbg-ACHPA-OH (938 mg, 100%).

B) Boc-Tbg-ACHPA-Glu(OBzl)-NHCH₂CH₂Ph

By following the procedure of section A) of example 3, but replacing Boc-ACHPA-OH with Boc-Tbg-ACHPA-OH, then Boc-Tbg-ACHPA-Glu(OBzl)-NHCH₂CH₂Ph was obtained in a quantitative yield.

C) Title compounds of this example

The product of section B) of this example was subjected to hydrogenolysis according to the procedure of section D) of example 3 to give Ac-Tbg-ACHPA-Glu-NHCH₂CH₂Ph. ¹H NMR (CDCl₃ + MeOH-d₄) δ 1.1 (9H, s), 1.1-2.0 (22H, m), 2.1 (3H, s), 2.35 (4H, t), 2.8 (2H, t), 3.9 (1H, m), 4.0 (1H, m), 4.1 (1H, m), 4.4 (1H, m), 7.1-7.3 (5H, m), 8.0 (1H, m). Mass spectrum: 603 (M+H)⁺. Amino acid analysis: Glu, 1.00; Tbg, 0.88; ACHPA, 1.06.

The latter product (57 mg, 0.095 mmol) was dissolved in EtOAc (10 mL). A slight excess of diazomethane in dry Et₂O was added to the solution (as indicated by the mixture maintaining a yellow color). The mixture was evaporated to dryness. Trituration of the residue with Et₂O afforded Ac-Tbg-ACHPA-Glu(OMe)-NHCH₂CH₂Ph as a white solid (31.5 mg, 54%). Mass spectrum: 617 (M+N)⁺. Amino acid analysis: Glu, 1.00; Tbg, 1.03; ACHPA, 0.83.

By following the procedure of examples 3 or 4 but substituting the appropriate acid of formula X-OH wherein X is as defined herein, and/or substituting the appropriate amino acid derivatives and/or substituting the appropriate protected fragment of formula H-C''-Y wherein C'' and Y are as defined herein, the following compounds of formula 1 were obtained:

Example 5: (S)-HOCH₂CH(OH)C(O)-Tbg-ACHPA-Glu-NHCH₂CH₂Ph mass spectrum: 649 (M+H)⁺, amino acid analysis: Glu, 1.00; Tbg, 0.99; ACHPA, 0.75.

Example 6: (S)-HOCH₂CH(OH)C(O)-Tbg-ACHPA-Glu(OMe)NHCH₂CH₂Ph, mass spectrum: 663 (M+H)⁺, amino acid analysis: Glu, 1.15; Tbg, 0.89; ACHPA, 0.96.

Example 7: CH₃CH₂C(O)-Tbg-ACHPA-Glu-NHCH₂CH₂Ph, mass spectrum: 617 (M+H)⁺, amino acid

10

analysis: Glu, 1.00; Tbg, 0.81; ACHPA, 0.80.

Example 8: $(CH_3)_3CCH_2C(O)$-Tbg-ACHPA-Glu-NHCH$_2$CH$_2$Ph, mass spectrum: 659 (M + N)$^+$, amino acid analysis: Glu, 1.00; Tbg, 0.70; ACHPA, 0.83.

Example 9: Ac-Tbg-ACHPA-Cpa-NHCH$_2$CH$_2$Ph mass spectrum: 585 (M + H)$^+$, amino acid analysis: Tbg, 0.92; ACHPA, 0.86; Cpa, 0.90.

Example 10: $(C_2H_5)_2$CHC(O)-Val-ACHPA-Cpa-NHCH$_2$CH$_2$Ph, mass spectrum: 641 (M + H)$^+$, amino acid analysis: Val, 1.00; ACHPA, 1.03; Cpa, 1.00.

Likewise, by following the procedures of examples 3 or 4 with the appropriate replacements, the following compounds of formula 1 can be prepared:

CH$_3$CH$_2$C(O)-Val-ACHPA-Glu-OCH$_2$CH$_2$Ph

HOCH$_2$CH$_2$C(O)-Ile-Sta-Cpa-OCH$_3$

HOCH$_2$CH$_2$C(O)-Ile-ACHPA-Glu-NHCH$_2$CH$_2$CH$_2$-(cyclohexyl)

(S)-HOCH$_2$CH(OH)C(O)-Tbg-ACHPA-Glu-NH-(cyclopropyl)

(R)-CH$_3$CH(OH)CH$_2$C(O)-Leu-ACCPA-Adp-NHCH$_2$CH$_2$Ph

Ac-Ala-AHPPA-Glu-OCH$_2$CH$_2$CH$_2$-(4F-Ph)

Ac-Ala-ACHPA-GluΨ[CSNH]-NHCH$_2$CH$_2$CH$_2$Ph

## Example 11

Ac-Val-PheΨ[CH$_2$NH]Leu-Glu-NHCH$_2$CH$_2$Ph

A) H-PheΨ[CH$_2$NH]Leu-OH p-nitrobenzyl ester hydrochloride

Sodium cyanoborohydride (2.83 g. 45.0 mmol) was added portionwise over 15 min to a stirred solution of H-Leu-OH p-nitrobenzyl ester hydrochloride (10.36 g, 36.3 mmol) and Boc-phenylalaninal (15.06 g, 70.0 mmol), described by D.H. Rich and E.T.O. Sun, supra, in MeOH (250 mL). The resulting mixture was stirred for 18 h. The reaction mixture was added dropwise to a stirred, ice-cold saturated aqueous solution of NaHCO$_3$ (2.5 L). The aqueous solution was extracted twice with EtOAc. The combined organic extracts were washed three times with a saturated aqueous solution of NaCl. The organic extract was dried (MgSO$_4$) and concentrated. The residue was triturated with hexane to give a solid. The solid was purified by chromatography on silica gel (eluent: EtOAc-hexane, 1:4) to give Boc-PheΨ[CH$_2$NH]Leu-OH p-nitrobenzyl ester as a white solid (12.33 g, 76%). $^1$H NMR (CDCl$_3$) 0.85-0.95 (6H, m), 1.3-1.85 (12H, m), 2.3-2.75 (2H, m), 2.75-2.9 (2H, m), 3.3 (2H, t), 3.7-3.9 (1H, m), 4.5-4.7 (1H, m), 5.2 (2H, s), 7.1-7.4 (5H, m), 7.45-8.25 (4H, 2d).

A solution of the latter product (11.62 g, 25.9 mmol) in 8N HCl/dioxane (90 mL) was stirred at room temperature under a nitrogen atmosphere for 30 min. The solvent was evaporated and the residue was dried in high vacuum to give H-PheΨ[CH$_2$NH]Leu-OH p-nitrobenzyl ester hydrochloride (10.85 g, 100%).

B) H-Val-PheΨ[CH$_2$NH]Leu-OH p-nitrobenzyl ester

A solution of the latter compound (5.0 g, 12.0 mmol), i.e. the first reactant, Boc-Val-OH (2.86 g, 13.2 mmol), i.e. the second reactant, and N-methylmorpholine (3.95 ml, 36 mmol) in DMF (50 mL) was adjusted to pH 8 (wet pH paper) by adding additional N-methylmorpholine. BOP (6.36 g, 14.4 mmol) was added to the solution. After 20 min, the pH was again adjusted to pH 8. The resultant mixture was stirred 18 h at room temperature under a nitrogen atmosphere. Thereafter, the mixture was diluted with Et$_2$O. The diluted mixture was washed successively with H$_2$O, a saturated aqueous solution of NaHCO$_3$ (three times) and a saturated aqueous solution of NaCl, dried (Na$_2$SO$_4$) and concentrated to dryness. Chromatography of the residue on silica gel [eluent: EtOAc/hexane (1:3)] gave Boc-Val-PheΨ[CH$_2$NH]Leu-OH p-nitrobenzyl ester (4.8 g, 69%).

A solution of the latter compound (1.0 g, 1.7 mmol) in 8N HCl/dioxane (15 mL) was stirred at room temperature for 30 min under a nitrogen atmosphere. The solvent was evaporated and the residue was dried in high vacuum to give H-Val-PheΨ[CH$_2$NH]Leu-OH p-nitrobenzyl ester hydrochloride (0.89 g, 100%).

C) Ac-Val-PheΨ[CH$_2$NH]Leu-OH

By following the coupling procedure described in the first paragraph of section B) of this example but using H-Val-PheΨ-[CH$_2$NH]Leu-OH p-nitrobenzyl ester hydrochloride as the first reactant and using acetic acid as the second reactant, then Ac-Val-PheΨ[CH$_2$NH]Leu-OH p-nitrobenzyl ester was obtained as a white solid (73% yield) after trituration of the crude product with Et$_2$O. (Purification by chromatography was not required.)

The latter compound (392 mg, 0.70 mmol) was subjected to hydrogenolysis using 10% Pd/C as catalyst according to the procedure of section D) of example 3 to give Ac-Val-PheΨ[CH$_2$NH]Leu-OH as a white solid (241 mg, 81%) after trituration of the crude product with Et$_2$O.

D) Ac-Val-PheΨ[CH$_2$NH]Leu-Glu(OBzl)-NHCH$_2$CH$_2$Ph

By following the coupling procedure described in the first paragraph of section B) of this example but using Ac-Val-PheΨ-[CH$_2$NH]Leu-OH as the first reactant and using H-Glu-(OBzl)NH CH$_2$CH$_2$Ph as the second reactant, then Ac-Val-PheΨ[CH$_2$NH]Leu-Glu(OBzl)NHCH$_2$CH$_2$Ph was obtained as a white solid (98% yield) after trituration of the crude product with hexane. (Purification by chromatography was not required.)

E) Title compound

The latter product (46 mg, 0.65 mmol) was subjected to hydrogenolysis using 10% Pd/C as catalyst according to the procedure of section D) of example 3 to give Ac-Val-PheΨ[CH$_2$NH]Leu-Glu-NHCH$_2$CH$_2$Ph as a white solid (29 mg, 73%) after trituration with Et$_2$O. $^1$H NMR (MeOH-d$_4$) δ 0.95-1.07 (12H, m), 1.55-1.64 (2H, m), 1.79-1.86 (2H, m), 2.08 (6H, s), 2.34-2.41 (2H, m), 2.79-3.11 (6H, m), 3.32-3.36 (1H, m), 3.5-3.6 (1H, m), 4.12 (1H, d), 4.2-4.4 (1H, m), 4.4-4.8 (1H, m), 7.23-7.42 (10H, m). Mass spectrum: 638 (M + H)$^+$. Amino acid analysis: Glu, 1.00; Val, 0.65; (PheΨ[CH$_2$NH]Leu does not hydrolyze and Val-PheΨ[CH$_2$NH]Leu only partially hydrolyzes).

Analogous compounds of formula 1 in which B is NHCH(R$^2$)CH$_2$NH(R$^3$)C(O) can be prepared by selecting the appropriate reactants and following the procedure of this example. Example of such compounds of formula 1 are:

Ac-Tbg-PheΨ[CH$_2$NH]Leu-Glu-NHCH$_2$CH$_2$CH$_2$Ph
Ac-Tbg-PheΨ[CH$_2$NH]Cpa-Glu-OCH$_2$CH$_2$Ph
(C$_2$H$_5$)$_2$CHC(O)-LeuΨ[CH$_2$NH]Phe-Adp-OCH$_2$CH$_2$Ph
(S)-HOCH$_2$CH(OH)C(O)-TyrΨ[CH$_2$NH]Cha-Glu(OMe)-NHCH$_2$CH$_2$Ph
CH$_3$CH$_2$C(O)-PheΨ[CH$_2$NH]Leu-NHCH[CH$_2$CH(CH$_3$)COOH]C(O)-NHCH$_2$CH$_2$Ph
HOCH$_2$CH$_2$C(O)-PheΨ[CH$_2$NH]Leu-GluΨ[CSNH]-NH(CH$_2$)$_5$CH$_3$
HOCH$_2$CH$_2$C(O)-PheΨ[CH$_2$NH]Cpa-Glu-OCH$_2$CH$_2$CH$_2$-(cyclohexyl)

Example 12
Recombinant HIV Protease HPLC Assay:
Enzyme: HIV protease was expressed in E. coli [construct pBRT1 prt$^+$, see W.G. Farmerie et al., Science, 236,305 (1987)], according to the following protocol:
Unless stated otherwise, all solutions are aqueous solutions.

(i) Fermentation

E. coli cells containing the pBRT1 prt$^+$ plasmid were used to inoculate a seed culture media composed of Luria-Bertani Broth containing 100 μg/mL of ampicillin. Flasks were incubated at 37° under agitation for 17 h. Production flasks containing sterile M9 broth, supplemented with 100 μg/ml of ampicillin, were inoculated at a level of 1% (v/v) using the seed culture described above. Total volume in each production flask was 500 mL in a 2 L Erlenmeyer flask. Flasks were incubated at 37° under agitation until a cell concentration corresponding to an optical density (λ = 540 m) of 0.6 was reached (no dilution). This time span was routinely 3-4 h. Flasks were then supplemented with 5mM isopropylthiogalactoside (IPTG, Research Organics, Cleveland, Ohio, USA) and incubation was continued until the cell concentration reached an optical density of 0.2 at 16-fold dilution. Flasks were then supplemented with 1mM phenylmethylsulfonyl fluoride (PMSF) and rapidly chilled to 4°. The bacterial cells were recovered by centrifugation at 4°. The wet pellet was stored at -70°.

(ii) <u>Extraction and Preparation of Assay Grade Enzyme:</u>

All steps below were performed at 4° unless otherwise indicated. Thawed cells were mixed with buffer A [50 mM tris(hydroxymethyl)aminoethane HCl(Tris-HCl, pH 7.4); 0.6 mM ethylenediaminetetraacetic acid (EDTA); 0.375 M NaCl, 0.2% Nonidet P-40 [R] (BDH Chemicals Ltd., Poole, UK); 1mM PMSF], at a ratio of one part cells to nine parts buffer A. Diatomaceous earth (Celite 545 [R], John Manville, Lompoc, CA, USA) was added at a ratio of two parts to one part of wet cell weight. The resulting slurry was homogenized at high speed (ca. 20,000 rpm) on a Waring [R] industrial blender for 8 x 15 sec. pulses. Cell debris/Celite [R] was collected by centrifugation and the resulting pellet was extracted with 4.5 parts of buffer A to one part wet solids using the homogenization procedure described above. Supernatants from both homogenization steps were combined and soluble protein was precipitated by the addition of solid $(NH_4)_2SO_4$ to yield a final concentration of 75% saturation. This mixture was agitated for 60 min and the precipitate was recovered by centrifugation. The resulting pellet was suspended in buffer B [50mM Tris-HCl, pH 8; 30 mM NaCl; 1 mM DL-dithiothreitol(DTT); 1 mM EDTA; 1 mM PMSF; 10% glycerol], and dialyzed for 18h against the same buffer.

An aliquot of the dialyzed extract containing 150 mg of the protein was loaded onto a Sephadex A25 [R] anion exchange column. (Pharmacia, Uppsala, Sweden) with bed dimensions of 70 cm length and 2.5 cm diameter. The sample was eluted isocratically with buffer B at a linear flow rate of 10 cm/h. Fractions containing HIV protease activity (see below for assay description) were combined, and soluble protein was precipitated by the addition of saturated aqueous $(NH_4)_2SO_4$ to yield a total $(NH_4)_2SO_4$ concentration of 85% saturation. Precipitated protein was removed by centrifugation and the resulting pellet was dissolved in buffer C[50 mM 2-(4-morpholino)ethanesulfonic acid (MES), pH 5.5; 150 mM NaCl; 1 mM DTT; 1 mM EDTA; 10% glycerol]. This preparation was dialyzed for 18h against buffer C, and then frozen at -70°. All crude extracts were purified by chromatography in aliquots containing 150 mg of protein in the same manner as described above. The final preparations from each batch were pooled, divided into 34µL aliquots and stored at -70°. The final protein recovered from a 20 L fermentation was typically 300 mg with a specific activity for HIV protease of 18.2 mmoles of substrate cleaved/min/mg.

The aliquots were diluted to 1/38 of the original concentration with buffer, see below, prior to use (i.e. the enzyme working solution).

Substrate: VSFNFPQITL-NH2, MW 1164, see Krausslich et al., Proc. Natl. Acad. Sci. USA, 86, 807 (1989), was used as substrate. The substrate was made into 10 mM stock in DMSO and stored at 4°. Prior to use, the stock was diluted with buffer to give a 400µM solution (i.e. the substrate working solution).

Buffer: MES (100 mM), KCl (300 mM) and EDTA (5 mM) were dissolved in distilled $H_2O$ (90 mL) and the solution was adjusted to pH 5.5 with concentrated aqueous NaOH. The latter solution was diluted to 100 mL with $H_2O$ to give the buffer.

Procedure: (1) The assay mixture was prepared by mixing 20µL of the substrate working solution, 10µL of the solution of the test compound in 10% DMSO and 10µL of the enzyme working solution. (2) The assay mixture was incubated at 37° for 30 min. (3) The reaction was quenched by adding 200µL of 2% aqueous TFA. (4) The substrate and products (i.e. VSFNF and PQITL-NH2) were separated by subjecting 100µL of the quenched assay mixture to HPLC using a Perkin-Elmer 3 x 3 CRC8 column (Perkin Elmer Inc., Norwalk, CT, USA) with a stepwise gradient at a flow rate of 4 mL/min. The gradient is as follows:

    0 - 0.5 minutes, 70% A / 30% B;
    0.5 - 3.0 minutes, 67% A / 33% B;
    3.0 - 5.0 minutes, 20% A / 80% B;
    5.0 - 6.5 minutes, 70% A / 30% B;

where A is 3 mM sodium dodecyl sulfate / 0.05% $H_3PO_4$ in $H_2O$ and B is 0.05% $H_3PO_4$ in acetonitrile. Elution was monitored at 210 nm. (5) A control, which was the assay mixture without the test compound, was subjected simultaneously to steps 2 to 4.

Inhibition Studies: Cleavage products and remaining parent substrate were quantified by either peak height or by integration of the appropriate HPLC peaks. Substrate conversion was calculated using the following relationship:

% Conversion = $\dfrac{\text{Sum of peak height or peak area of products}}{\text{Sum of peak height or peak}} \times 100$

area of substrate and products

Enzyme inhibition of the test compound was calculated as follows:

% Inhibition = 100 -    % Conversion for assay mixture X 100 % Conversion of control

The concentration of the test compound which causes a 50% inhibition of the HIV-protease, i.e. the $IC_{50}$, was determined as follows: The percent inhibition of the enzyme was determined for a minimum of three different concentrations of the test compound. Thereafter, the $IC_{50}$ was determined graphically by plotting the percent inhibition of the enzyme against the concentration of the test compound.

The following table of exemplified compounds of formula 1 lists their $IC_{50}$ as determined in the recombinant HIV protease HPLC assay.

| Compound | Example in which compound is prepared | IC$_{50}$ (nM) |
|---|---|---|
| Ac-Val-ACHPA-Glu-NHCH$_2$CH$_2$Ph | 3 | 31 |
| Ac-Val-ACHPA-Glu(OMe)-NHCH$_2$CH$_2$Ph | 3 | 480 |
| Ac-Tbg-ACHPA-Glu-NHCH$_2$CH$_2$Ph | 4 | 12 |
| Ac-Tbg-ACHPA-Glu(OMe)-NHCH$_2$CH$_2$Ph | 4 | 150 |
| (S)-HOCH$_2$CH(OH)C(O)-Tbg-ACHPA-Glu-NHCH$_2$CH$_2$Ph | 5 | 9.2 |
| (S)-HOCH$_2$CH(OH)C(O)-Tbg-ACHPA-Glu(OMe)-NHCH$_2$CH$_2$Ph | 6 | 200 |
| CH$_3$CH$_2$C(O)-Tbg-ACHPA-Glu-NHCH$_2$CH$_2$Ph | 7 | 15 |
| (CH$_3$)$_3$CCH$_2$C(O)-Tbg-ACHPA-Glu-NHCH$_2$CH$_2$Ph | 8 | 110 |
| Ac-Tbg-ACHPA-Cpa-NHCH$_2$CH$_2$Ph | 9 | 93 |
| (C$_2$H$_5$)$_2$CHC(O)-Val-ACHPA-Cpa-NHCH$_2$CH$_2$CH$_2$Ph | 10 | 135 |
| Ac-Val-Pheψ[CH$_2$NH]Leu-Glu-NHCH$_2$CH$_2$Ph | 11 | 16 |

Example 13

The following protocol, used for screening antiviral effects of the compounds of formula 1, is adapted from a plaque assay utilizing HTLV-I transformed cells which was developed by Harada et al., supra. HTLV-I transformed cells are used in these assays because of the rapidity with which HIV will replicate in these

15

cells.

1. The test compound is dissolved in dimethylsulfoxide to a concentration of 5 mg/mL. The resultant solution can be stored at 4° until used.

2. The resultant solution is diluted in RPMI 1640 (Gibco Laboratories, St. Lawrence, MA, USA) to four times (4x) the final concentration which is to be tested. Once diluted in RPMI 1640, the solution is used in the cell culture assay within 4 h.

3. The 4x solution ($50 \mu L$) is added to triplicate wells of a 96 well flat bottomed microtiter plate. RPMI 50 L also is added to control wells.

4. C8166 cells ($5 \times 10^{-4}$) in $50 \mu L$ of HEPES-buffered RPMI 1640, 10% heat inactivated fetal calf serum (FCS), $12.5 \mu L/ml$ gentamicin (complete media) are added to all wells.

5. Fifty times $TCID_{50}$ of H9/HTLV-IIIB stock (stored in liquid nitrogen as cell culture supernatant in 50% FCS) in $100 \mu L$ of complete media is added to all wells. Infectious titer of virus stocks are as previously determined by end point dilution on C8166 cells. Titer of stocks are stable for 6-12 months when stored at -193°.

6. Microtiter plates are then placed on level shelves of a 37°, 5% $CO_2$ humidified incubator for 72 h.

7. Plates are then removed and centers of syncytia are counted in each well by low power phase contrast light microscopy. Each cluster of cells which shows evidence of any syncytia formation is counted as one center of syncytia. Control wells should have between 25 and 75 centers of syncytia per well.

8. Percent inhibition of syncytia formation is calculated by the formula:

$$\% \text{ inhibition} = 100 \times \frac{\left(\begin{array}{c}\text{\# syncytial centers} \\ \text{in control wells}\end{array}\right) - \left(\begin{array}{c}\text{\# syncytial centers in} \\ \text{test wells}\end{array}\right)}{\begin{array}{c}\text{\# syncytial center in} \\ \text{control wells}\end{array}}$$

When tested according to this procedure, $(S)\text{-HOCH}_2\text{CH(OH)C(O)-Tbg-ACHPA-Glu(OMe)NHCH}_2\text{CH}_2\text{PH}$ of example 6 and $CH_3\text{CH}_2\text{C(O)-Tbg-ACHPA-Glu-NHCH}_2\text{CH}_2\text{Ph}$ of example 7 exhibited 100% and 49% inhibition at $20 \mu g/mL$.

**Claims**

1. A compound of formula 1

   X-A-B-C'-Y    (1)

   wherein X is
   (i) a lower alkanoyl,
   (ii) a lower alkanoyl monosubstituted with a hydroxy, or
   (iii) an alkanoyl, containing three to six carbon atoms, disubstituted with hydroxy, each hydroxy being on different carbon atoms thereof,
   A is $NHCH(R^1)C(O)$ wherein $R^1$ is lower alkyl,
   B is $NHCH(R^2)$-Q wherein $R^2$ is
   (i) lower alkyl,
   (ii) lower cycloalkyl,
   (iii) (lower cycloalkyl)methyl,
   (iv) phenyl or phenyl monosubstituted with hydroxy, lower alkyl or lower alkoxy, or
   (v) benzyl or benzyl monosubstituted on the aromatic portion thereof with hydroxy, halo, lower alkyl or lower alkoxy, and
   Q is $CH(OH)CH_2C(O)$ or $CH_2NHCH(R^3)C(O)$ wherein $R^3$ has the same meaning as defined hereinabove for $R^2$;
   C' is $NHCH(R^4)C(W)$ wherein $R^4$ is (lower cycloalkyl)methyl, $CR^5R^6CR^7R^8C(O)$-T or $CR^5R^6CR^7R^8CR^9R^{10}C(O)$-T wherein $R^5$ to $R^{10}$, inclusive, are each independently a hydrogen or lower alkyl and T is hydroxy or lower alkoxy, and W is oxo or thioxo;

Y is $OR^{11}$ wherein $R^{11}$ is

(i) (1-10C)alkyl,

(ii) lower cycloalkyl,

(iii) (lower cycloalkyl)-(lower alkyl), or (iv)phenyl(lower alkyl) or phenyl(lower alkyl) monosubstituted on the aromatic portion thereof with hydroxy, lower alkyl, lower alkoxy, halo, $SO_2NH_2$ or $S(O)_nR^{12}$ wherein n is an integer from zero to two and $R^{12}$ is lower alkyl; or

Y is $NR^{13}R^{14}$ wherein $R^{13}$ is hydrogen or lower alkyl and $R^{14}$ has the same meaning as defined hereinabove for $R^{11}$; or a therapeutically acceptable salt thereof.

2. A compound as recited in claim 1 wherein

X is as defined in claim 1;

A is Ala, Abu, Val, Nva, Leu, Ile, Nle or Tbg;

B is $NHCH(R^2)$-Q wherein $R^2$ is lower alkyl, (lower cycloalkyl)methyl, benzyl, (4-hydroxyphenyl)-methyl or
(4-methoxyphenyl)methyl and Q is $CH(OH)CH_2C(O)$ or $CH_2NHCH(R^3)C(O)$ wherein $R^3$ is lower alkyl, (lower cycloalkyl)methyl or phenyl;

C' is $NHCH(R^4)C(W)$ wherein $R^4$ is cyclopropylmethyl, $CH_2CH_2C(O)OH$, $CH_2CH(CH_3)C(O)OH$, $CH-(C_2H_5)CH_2C(O)OH$, $CH_2CH_2C(O)OCH_3$, $CH_2CH(CH_3)C(O)OCH_3$, $CH(CH_3)CH(CH_3)C(O)OCH_3$, $CH_2CH_2CH_2C(O)OH$ or $CH_2CH_2CH_2C(O)OCH_3$ and W is oxo or thioxo; and

Y is $OR^{11}$ wherein $R^{11}$ is

(i) (1-10C)alkyl,

(ii) cyclopropyl, cyclopentyl or cyclohexyl,

(iii) (lower cycloalkyl)methyl, (lower cycloalkyl)ethyl or (lower cycloalkyl)propyl,

(iv) phenyl(lower alkyl) or phenyl(lower alkyl) substituted at position 4 of the phenyl with hydroxy, lower alkoxy, fluoro, chloro or lower alkylthio; or

Y is $NR^{13}R^{14}$ wherein $R^{13}$ is hydrogen or methyl and $R^{14}$ has the same meaning as defined in this claim for $R^{11}$;

or a therapeutically acceptable salt thereof.

3. A compound as recited in claim 1 wherein X is acetyl, 1-oxopropyl, 1-oxobutyl, 3-methyl-1-oxobutyl, 3,3-dimethyl-1-oxobutyl, 2-ethyl-1-oxobutyl, 2-hydroxy-1-oxopropyl, 3-hydroxy-1-oxopropyl or 2,3-dihydroxy-1-oxopropyl; A is Val, Nva, Leu, Ile, Nle or Tbg; B is $NHCH(R^2)CH(OH)CH_2C(O)$ wherein $R^2$ is 1-methylpropyl, 2-methylpropyl, 1,1-dimethylethyl, cyclopropylmethyl, cyclohexylmethyl, benzyl, (4-hydroxyphenyl)methyl or (4-methoxyphenyl)methyl, or B is $NHCH(R^2)CH_2NHCH(R^3)C(O)$ wherein $R^2$ is as defined in the last instance and $R^3$ is 1-methylethyl, 1-methylpropyl, 2-methylpropyl, 1,1-dimethylethyl, 2,2-dimethylpropyl, cyclopropylmethyl, cyclohexylmethyl or phenyl; C' is Cpa, Glu, Glu-(OMe), Adp or Adp(OMe); and Y is methoxy, ethoxy, hexyloxy, octyloxy, cyclopropyloxy, 3-cyclopropylpropoxy, 2-cyclohexylethoxy, 3-cyclohexylpropoxy, 2-phenylethoxy, 2-(4-fluorophenyl)ethoxy, 2-[4-(methylthio)phenyl]ethoxy, 3-phenylpropoxy, 3-(4-hydroxyphenyl)propoxy, 3-(4-propoxyphenyl)propoxy, 3-(4-fluorophenyl)propoxy, 3-(4-chlorophenyl)propoxy, 3-[4-(methylthio)phenyl]propoxy, methylamino, 3-cyclopropylpropylamino, 2-cyclohexylethylamino, 3-cyclohexylpropylamino, 2-phenylethylamino, 2-(4-fluorophenyl)ethylamino, 2-[4-(methylthio)phenyl]ethylamino, 3-phenylpropylamino, 3-(4-hydroxyphenyl)propylamino, 3-(4-methoxyphenyl)propylamino, 3-(4-propoxyphenyl)propylamino, 3-(4-fluorophenyl)propylamino, 3-(4-chlorophenyl)propylamino or 3-[4(methylthio)phenyl]propylamino; or a therapeutically acceptable salt thereof.

4. A compound as recited in claim 1 wherein X is acetyl, 1-oxopropyl, 3,3-dimethyl-1-oxobutyl, 2-ethyl-1-oxobutyl, 2(S)-hydroxy-1-oxopropyl or 2(S)-3-dihydroxy-1-oxopropyl; A is Val or Tbg, B either is $NHCH-(R^2)CH(OH)CH_2C(O)$ wherein $R^2$ is 1-methylpropyl, 2-methylpropyl, butyl, cyclopropylmethyl or cyclohexylmethyl, or B is $NHCH(R^2)CH_2NHCH(R^3)C(O)$ wherein $R^2$ is benzyl and $R^3$ is 2-methylpropyl or cyclopropylmethyl, the two asymmetric centers of the last said radical having the (S) chiral configuration; C' is Cpa, Glu or Glu(OMe) and Y is 2-phenylethoxy, 3-phenylpropoxy, 2-phenylethylamino, 2-(4-fluorophenyl)ethylamino, 2-[4-(methylthio)phenyl]ethylamino, 3-phenylpropylamino, 3-(4-hydroxyphenyl)propylamino, 3-(4-propoxyphenyl)propylamino or 3-[4-(methylthio)-phenyl]propylamino; or a therapeutically acceptable salt thereof.

5. A compound as recited in claim 1 selected from the group consisting of Ac-Val-ACHPA-Glu-NHCH$_2$CH$_2$Ph, Ac-Val-ACHPA-Glu(OMe)-NHCH$_2$CH$_2$Ph, Ac-Tbg-ACHPA-Glu-NHCH$_2$CH$_2$Ph, Ac-Tbg-ACHPA-Glu(OMe)-NHCH$_2$CH$_2$Ph, (S)-HOCH$_2$CH(OH)C(O)-Tbg-ACHPA-Glu-CH$_2$CH$_2$Ph, (S)-HOCH$_2$CH-(OH)C(O)-Tbg-ACHPA-Glu(OMe)-NHCH$_2$CH$_2$Ph, CH$_3$CH$_2$C(O)-Tbg-ACHPA-Glu-NHCH$_2$CH$_2$Ph, (CH$_3$)$_3$CCH$_2$C(O)-g-ACHPA-Glu-NHCH$_2$CH$_2$Ph, Ac-Tbg-ACHPA-Cpa-NHCH$_2$CH$_2$Ph, (C$_2$H$_5$)$_2$CHC(O)-Val-ACHPA-Cpa-NHCH$_2$CH$_2$CH$_2$Ph and Ac-Val-PheΨ[CH$_2$NH]Leu-Glu-NHCH$_2$CH$_2$Ph.

6. A pharmaceutical composition comprising a compound as recited in claim 1, or a therapeutically acceptable salt thereof, and a pharmaceutically acceptable carrier.

7. The use of a compound as recited in anyone of claims 1 to 5, or a therapeutically acceptable salt thereof, for the manufacture of a medicament for treating HIV infections in a human.

8. The use of a compound as recited in anyone of claims 1 to 5, or a therapeutically acceptable salt thereof, for the manufacture of a medicament for protecting human cells against HIV pathogenesis.

9. A process for preparing a compound of formula 1 as recited in claim 1 comprising:
   coupling the acid of formula X-OH wherein x is as defined in claim 1 with an intermediate of formula H-A-B-C''-Y wherein A, B and Y are as defined in claim 1 and C'' has the same meaning as defined for C'' in claim 1 except that a side chain carboxy or ester group is replaced by a protected carboxy to obtain:
   a) when the intermediate does not bear a protected carboxy, the corresponding compound of formula 1; or
   b) when the intermediate bears a protected carboxy, the corresponding intermediate of formula X-A-B-C''-Y and subjecting the last said intermediate to carboxy deprotection, or carboxy deprotection and esterification, to obtain the corresponding compound of formula 1;
   and, if desired transforming the compound of formula 1 into a therapeutically acceptable salt.

18